# EUROPEAN PATENT SPECIFICATION

(11) **EP 4 686 465 B1**
(45) Date of publication and mention of the grant of the patent: **14.10.2026**
(21) Application number: 24213478.1
(22) Date of filing: 18.11.2024
(51) Int. Cl.: A61K 8/34, A61K 8/37, A61K 8/39, A61K 8/44, A61K 8/49, A61K 8/67, A61K 8/86, A61Q 19/02

(54) **GLABRIDIN COMPOSITION WITH HIGH SKIN PERMEABILITY, AND PREPARATION METHOD AND USE THEREOF**
GLABRIDINZUSAMMENSETZUNG MIT HOHER HAUTDURCHLÄSSIGKEIT SOWIE HERSTELLUNGSVERFAHREN UND VERWENDUNG DAVON
COMPOSITION DE GLABRIDINE À HAUTE PERMÉABILITÉ CUTANÉE, SON PROCÉDÉ DE PRÉPARATION ET SON UTILISATION

(30) Priority: 31.07.2024 CN 202411035288
(43) Date of publication of application: 04.02.2026
(73) Proprietor: Guangzhou Fanzhirong Cosmetics Co., Ltd., Guangzhou Guangdong 510700 (CN); Guangzhou Qingnang Biotechnology Co., Ltd, Guangzhou Guangdong 510700 (CN)
(72) Inventor: LIU, Haiyan, Guangzhou, 510700 (CN); HOU, Sen, Guangzhou, 510700 (CN); LI, Anzhang, Guangzhou, 510700 (CN); YANG, Tuzhen, Guangzhou, 510700 (CN); WEI, Jie, Guangzhou, 510700 (CN); WANG, Anning, Guangzhou, 510700 (CN)
(74) Representative: Metida

(56) References cited:
- CN-A- 113 893 191
- CN-A- 113 908 076
- CN-B- 109 549 864
- CN-B- 113 476 340
- DATABASE GNPD [online] MINTEL; 12 March 2024 (2024-03-12), ANONYMOUS: "Collagen Essence Lotion", XP093271627, retrieved from https://www.gnpd.com/sinatra/recordpage/11526964/ Database accession no. 11526964
- DATABASE GNPD [online] MINTEL; 1 September 2023 (2023-09-01), ANONYMOUS: "Essence Cream", XP093244414, retrieved from https://www.gnpd.com/sinatra/recordpage/11036326/ Database accession no. 11036326

## Description

### TECHNICAL FIELD

The present application relates to the field of cosmetics, in particular to a glabridin composition with high skin permeability and a preparation method and use thereof.

### BACKGROUND

Glabridin, commonly known as "whitening gold", can effectively inhibit tyrosinase activity, dopachrome tautomerism, DHICA oxidase activity and cytochrome P450 3A4 enzyme (CYP3A4 for short) activity, thus achieving the purpose of whitening and spot removing efficiently and green. However, glabridin's insolubility in water and low permeability properties limit its application in formulas.

Glabridin is a fat-soluble active substance, which is soluble in polyols, slightly soluble in oils, and insoluble in water. Therefore, in order to enhance the skin penetration ability of glabridin, it is necessary to not only solve the problem of glabridin solubility, but also use carrier technologies such as microencapsulation, liposome and nano-emulsion to wrap the active ingredients, and carry glabridin to penetrate the stratum corneum of skin to enhance the permeability. However, according to the conventional preparation method and formula composition, there are technical problems such as low skin permeation rate, low glabridin solubility, poor stability, high irritation, high preparation cost and complicated operation.

The prior art provides methods for cyclodextrin inclusion of glabridin, such as preparing cyclodextrin inclusion complexes by technologies such as precipitation methods, grinding methods, freeze drying methods, and spray drying methods. These technologies require common equipment for non-cosmetic production, and the molecular weight of cyclodextrin is large and its outside is hydrophilic, thus greatly weakening the ability of active substances to directly penetrate the hydrophobic stratum corneum. In addition, in skin care product formulas, the cyclodextrin encapsulating system still has two disadvantages. One is that cyclodextrin itself is very sticky, which will make consumers feel an unpleasant sticky feeling during use. The other is that the cyclodextrin encapsulating system is susceptible to crystal precipitation in formulas. It is shown in some documents that polyols such as pentanediol and hexanediol will affect the encapsulating stability of cyclodextrin, which leads to difficulties for formulators in developing cosmetic formulas.

The prior art also provides methods for phospholipid inclusion of glabridin, which prepare phospholipid inclusion complexes by technologies such as film rehydration methods, reverse phase evaporation methods, injection methods, and microfluidic methods. These technologies also require common equipment for non-cosmetic production, and liposomes may aggregate, leak or change their structures during storage, resulting in the loss or early release of active substances, and phospholipases in vivo can decompose liposomes, which directly affects the delivery efficiency and controlled release properties of drugs.

CN109549864 B discloses an emulsion composition comprising glabridin, ethyl ascorbic acid and triglyceride oil, wherein the oil is eg. caprylic/capric triglyceride. The glabridin and ethyl ascorbic acid are added after the emulsion is formed. The examples show compositions comprising glabridin, caprylic/capric/succinic triglyceride, 1,3-butanediol, 3-o-ethyl ascorbic acid.

Therefore, there is still an urgent need for a glabridin composition with high skin permeation rate, good solubility, good stability, good skin feeling, low irritation, low preparation cost and simple operation process.

### SUMMARY OF THE INVENTION

### Overview of the invention

In order to provide a glabridin composition with high skin permeation rate, good solubility, no crystal precipitation during stable placement, good content stability, good pH stability, high mildness, good skin feeling, low preparation cost and simple operation process, the present application provides the following technical schemes.

In a first aspect, the present application provides a glabridin composition prepared from an S1 phase, an S2 phase and an S3 phase, in which the S1 phase consists of an oil and glabridin, the S2 phase is prepared from 3-o-ethyl ascorbic acid, an anionic surfactant, a nonionic surfactant, a polyol and water, and the S3 phase is water;
the nonionic surfactant is oleth-20 and polyglycerol-10 oleate; the anionic surfactant is sodium stearoyl glutamate; the oil is caprylic acid/capric acid triglyceride; the polyol is 1,3-butanediol;
based on a total mass of the glabridin composition, the glabridin has a content of 0.10-4.00 wt%, and the 3-o-ethyl ascorbic acid has a content of 0.10-4.00 wt%; the nonionic surfactant has a total content of 0.70 wt%-10.00 wt%, and the oil has a content of 1.00 wt%-40.00 wt%; the polyol has a content of 0.60 wt%-6.00 wt%, the anionic surfactant has a content of 0.10 wt%-1.00 wt%, water in the S2 phase has a content of 0.30 wt%-3.00 wt%, and the balance is the S3 phase;
in addition, the present application also provides a preparation method of the glabridin composition.

The adoption of the technical schemes of the present application is beneficial to enhancing the skin permeability and solubility of the glabridin composition, and enhancing the state stability, content stability, pH stability and mildness of the composition under high temperature and low temperature conditions, thus having unexpected technical effects.

The adoption of the preparation method provided by the present application does not need to use common equipment for non-cosmetic production, and only uses simple process operations. It is beneficial to enhance the skin permeability and solubility of the glabridin composition, and to enhance its state stability, content stability, pH stability and mildness under high temperature and low temperature conditions.

In a second aspect, the present application provides use of the glabridin composition described in the first aspect in preparation of cosmetics.

In a third aspect, the present application provides a cosmetic, including the glabridin composition described in the first aspect.

### Detailed description of the invention

In order to solve the above technical problems, the present application provides a glabridin composition and a preparation method thereof.

In a first aspect, the present application provides a glabridin composition.

A glabridin composition, which is prepared from an S1 phase, an S2 phase and an S3 phase, in which the S1 phase consists of an oil and glabridin, the S2 phase is prepared from 3-o-ethyl ascorbic acid, an anionic surfactant, a nonionic surfactant, a polyol and water, and the S3 phase is water;
the nonionic surfactant is oleth-20 and polyglycerol-10 oleate;
the anionic surfactant is sodium stearoyl glutamate;
the oil is caprylic acid/capric acid triglyceride;
the polyol is 1,3-butanediol;
based on a total mass of the glabridin composition, the glabridin has a content of 0.10-4.00 wt%, and the 3-o-ethyl ascorbic acid has a content of 0.10-4.00 wt%; the nonionic surfactant has a total content of 0.70 wt%-10.00 wt%, and the oil has a content of 1.00 wt%-40.00 wt%; the polyol has a content of 0.60 wt%-6.00 wt%, the anionic surfactant has a content of 0.10 wt%-1.00 wt%, water in the S2 phase has a content of 0.30 wt%-3.00 wt%, and the balance is the S3 phase;
a preparation method of the glabridin composition comprises the following steps:
   (1) mixing glabridin and the oil, heating for dissolution, and uniformly mixing to obtain the S1 phase;
   (2) mixing 3-o-ethyl ascorbic acid, the anionic surfactant, the nonionic surfactant, the polyol and water, heating for dissolution, and uniformly mixing to obtain the S2 phase;
   (3) adding the S1 phase obtained in the step (1) into the S2 phase obtained in the step (2) while stirring the S2 phase, and mixing, to form a gel; and
   (4) then mixing the gel obtained in the step (3) with the S3 phase to obtain the glabridin composition.

In some examples, the oleth-20 has a content of 0.10 wt%-4.00 wt%, and the polyglycerol-10 oleate has a content of 0.60 wt%-6.00 wt%, based on the total mass of the glabridin composition.

In some examples, based on the total mass of the glabridin composition, the glabridin has a content of 1.00-4.00 wt%, and the 3-o-ethyl ascorbic acid has a content of 1.00-4.00 wt%; the oleth-20 has a total content of 0.50 wt%-4.00 wt%, the polyglycerol-10 oleate has a content of 6.00 wt%, and the oil has a content of 10.00 wt%-40.00 wt%; the polyol has a content of 6.00 wt%, the anionic surfactant has a content of 1.00 wt%, water in the S2 phase has a content of 3.00 wt%, and the balance is the S3 phase.

In some examples, based on the total mass of the glabridin composition, the glabridin has a content of 0.10 wt%, and the 3-o-ethyl ascorbic acid has a content of 0.10 wt%; the oleth-20 has a total content of 0.10 wt%, the polyglycerol-10 oleate has a content of 0.60 wt%, and the oil has a content of 1.00 wt%; the polyol has a content of 0.60 wt%, the anionic surfactant has a content of 0.10 wt%, water in the S2 phase has a content of 0.30 wt%, and the balance is the S3 phase.

In some examples, based on the total mass of the glabridin composition, the glabridin has a content of 1.00 wt%, and the 3-o-ethyl ascorbic acid has a content of 1.00 wt%; the oleth-20 has a total content of 0.50 wt% wt%, the polyglycerol-10 oleate has a content of 6.00 wt%, and the oil has a content of 10.00 wt%; the polyol has a content of 6.00 wt%, the anionic surfactant has a content of 1.00 wt%, water in the S2 phase has a content of 3.00 wt%, and the balance is the S3 phase.

In some examples, based on the total mass of the glabridin composition, the glabridin has a content of 1.00 wt%, and the 3-o-ethyl ascorbic acid has a content of 1.00 wt%; the oleth-20 has a total content of 1.00 wt%, the polyglycerol-10 oleate has a content of 6.00 wt%, and the oil has a content of 10.00 wt%; the polyol has a content of 6.00 wt%, the anionic surfactant has a content of 1.00 wt%, water in the S2 phase has a content of 3.00 wt%, and the balance is the S3 phase.

In some examples, based on the total mass of the glabridin composition, the glabridin has a content of 1.00 wt%, and the 3-o-ethyl ascorbic acid has a content of 2.00 wt%; the oleth-20 has a total content of 0.50 wt%, the polyglycerol-10 oleate has a content of 6.00 wt%, and the oil has a content of 10.00 wt%; the polyol has a content of 6.00 wt%, the anionic surfactant has a content of 1.00 wt%, water in the S2 phase has a content of 3.00 wt%, and the balance is the S3 phase.

In some examples, based on the total mass of the glabridin composition, the glabridin has a content of 2.00 wt%, and the 3-o-ethyl ascorbic acid has a content of 2.00 wt%; the oleth-20 has a total content of 2.00 wt%, the polyglycerol-10 oleate has a content of 6.00 wt%, and the oil has a content of 20.00 wt%; the polyol has a content of 6.00 wt%, the anionic surfactant has a content of 1.00 wt%, water in the S2 phase has a content of 3.00 wt%, and the balance is the S3 phase.

In some examples, based on the total mass of the glabridin composition, the glabridin has a content of 4.00 wt%, and the 3-o-ethyl ascorbic acid has a content of 4.00 wt%; the oleth-20 has a total content of 4.00 wt%, the polyglycerol-10 oleate has a content of 6.00 wt%, and the oil has a content of 40.00 wt%; the polyol has a content of 6.00 wt%, the anionic surfactant has a content of 1.00 wt%, water in the S2 phase has a content of 3.00 wt%, and the balance is the S3 phase.

In some examples, the heating for dissolution in the step (1) is heating to 60°C-80°C for dissolution. In some examples, the heating for dissolution in the step (1) is heating to 60°C, 65°C, 70°C, 75°C or 80°C for dissolution.

In some examples, the heating for dissolution in the step (2) is heating to 60°C-70°C for dissolution. In some examples, the heating for dissolution in the step (2) is heating to 60°C, 65°C or 70°C for dissolution.

In some examples, the heating for dissolution in the step (1) is heating to 70°C for dissolution; and the heating for dissolution in the step (2) is heating to 60°C for dissolution.

In some examples, the feeding mass ratio of water to the polyol in the step (2) is 1:1-1:2.

In a second aspect, the present application provides use of the glabridin composition described in the first aspect.

Use of the glabridin composition described in the first aspect in preparation of cosmetics.

In a third aspect, the present application provides a cosmetic.

A cosmetic, including the glabridin composition described in the first aspect.

In some examples, the cosmetic is an essence dosage form.

In some examples, the cosmetic is an essential dosage form, and includes an A phase, a B phase, a C phase, a D phase, a E phase and an F phase; the A phase includes water, EDTA disodium and carbomer; the B phase includes p-hydroxyacetophenone, 1,2-pentanediol and 1,2-hexanediol; the C phase includes water and arginine; the D phase includes hydrogenated lecithin, ethoxydiglycol, palmitoyl tripeptide-8 and alpha-tocopherol; the E phase includes glutathione and an aqueous plant extract solution; and the F phase is the glabridin composition described in the first aspect.

In some examples, the aqueous plant extract solution is an aqueous peony flower solution.

In some examples, based on the total mass of the cosmetic, the F phase has a content of 1.000 wt%-2.000 wt%; glutathione in the E phase has a content of 0.001 wt%-0.050 wt%; the aqueous peony flower solution in the E phase has a content of 0.050 wt%-0.500 wt%; hydrogenated lecithin in the D phase has a content of 0.040-0.100 wt%; ethoxydiglycol in the D phase has a content of 0.010 wt%-0.100 wt%; palmitoyl tripeptide-8 in the D phase has a content of 0.001 wt%-0.005 wt%; alpha-tocopherol in the D phase has a content of 0.005 wt%-0.010 wt%; water in the C phase has a content of 1.000 wt%-2.000 wt%; arginine in the C phase has a content of 0.100 wt%-0.500 wt%; p-hydroxyacetophenone in the B phase has a content of 0.050 wt%-0.300 wt%; 1,2-pentanediol in the B phase has a content of 1.000 wt%-5.000 wt%; 1,2- hexanediol in the B phase has a content of 0.500 wt%-1.000 wt%; EDTA disodium in the A phase has a content of 0.020 wt%-0.100 wt%; carbomer in the A phase has a content of 0.100 wt%-0.500 wt%; and the balance is water in the A phase.

In some examples, based on the total mass of the cosmetic, the F phase has a content of 1.000 wt%; glutathione in the E phase has a content of 0.010 wt%; the aqueous peony flower solution in the E phase has a content of 0.100 wt%; hydrogenated lecithin in the D phase has a content of 0.060 wt%; ethoxydiglycol in the D phase has a content of 0.020 wt%; palmitoyl tripeptide-8 in the D phase has a content of 0.002 wt%; alpha-tocopherol in the D phase has a content of 0.005 wt%; water in the C phase has a content of 1.000 wt%; arginine in the C phase has a content of 0.200 wt%; p-hydroxyacetophenone in the B phase has a content of 0.100 wt%; 1,2-pentanediol in the B phase has a content of 3.000 wt%; 1,2- hexanediol in the B phase has a content of 0.500 wt%; EDTA disodium in the A phase has a content of 0.050 wt%; carbomer in the A phase has a content of 0.200 wt%; and the balance is water in the A phase.

In some examples, based on the total mass of the cosmetic, the F phase has a content of 2.000 wt%; glutathione in the E phase has a content of 0.050 wt%; the aqueous peony flower solution in the E phase has a content of 0.500 wt%; hydrogenated lecithin in the D phase has a content of 0.100 wt%; ethoxydiglycol in the D phase has a content of 0.100 wt%; palmitoyl tripeptide-8 in the D phase has a content of 0.005 wt%; alpha-tocopherol in the D phase has a content of 0.010 wt%; water in the C phase has a content of 2.000 wt%; arginine in the C phase has a content of 0.500 wt%; p-hydroxyacetophenone in the B phase has a content of 0.300 wt%; 1,2-pentanediol in the B phase has a content of 5.000 wt%; 1,2-hexanediol in the B phase has a content of 1.000 wt%; EDTA disodium in the A phase has a content of 0.100 wt%; carbomer in the A phase has a content of 0.500 wt%; and the balance is water in the A phase.

In some examples, based on the total mass of the cosmetic, the F phase has a content of 1.000 wt%; glutathione in the E phase has a content of 0.001 wt%; the aqueous peony flower solution in the E phase has a content of 0.050 wt%; hydrogenated lecithin in the D phase has a content of 0.040 wt%; ethoxydiglycol in the D phase has a content of 0.010 wt%; palmitoyl tripeptide-8 in the D phase has a content of 0.001 wt%; alpha-tocopherol in the D phase has a content of 0.005 wt%; water in the C phase has a content of 1.000 wt%; arginine in the C phase has a content of 0.100 wt%; p-hydroxyacetophenone in the B phase has a content of 0.050 wt%; 1,2-pentanediol in the B phase has a content of 1.000 wt%; 1,2- hexanediol in the B phase has a content of 0.500 wt%; EDTA disodium in the A phase has a content of 0.020 wt%; carbomer in the A phase has a content of 0.100 wt%; and the balance is water in the A phase.

In some examples, the cosmetic is an essence dosage form, and the preparation method of the cosmetic includes:
(i) mixing each component of the A phase, heating for dissolution, and then cooling to 40°C-50°C to obtain the A phase;
(ii) mixing each component of the B phase, heating for dissolution, and then cooling to normal temperature to obtain the B phase;
(iii) stirring each component of the C phase until they are completely dissolved to obtain the C phase;
(iv) mixing and stirring each component of the D phase, heating to 65°C-75°C, and homogenizing to obtain the D phase; and
(v) adding the B phase, the C phase, the D phase, the E phase and the F phase into the A phase obtained in the step (i) while stirring the A phase, and mixing, to obtain the cosmetic.

In some examples, the heating for dissolution in the step (i) is heating to 75-85°C for 5-40 minutes, and stirring until they are completely dissolved. In some examples, the heating for dissolution in the step (i) is heating to 75°C, 80°C or 85°C for 5 minutes, 10 minutes, 15 minutes, 20 minutes, 25 minutes, 30 minutes, 35 minutes or 40 minutes, and stirring until they are completely dissolved.

In some examples, the cooling in the step (i) is cooling to 40°C, 41°C, 42°C, 43°C, 44°C, 45°C, 46°C, 47°C, 48°C, 49°C or 50°C.

In some examples, the heating for dissolution in the step (ii) is heating to 60-70°C, and stirring until they are completely dissolved. In some examples, the heating for dissolution in the step (ii) is heating to 60°C, 61°C, 62°C, 63°C, 64°C, 65°C, 66°C, 67°C, 68°C, 69°C or 70°C, and stirring until they are completely dissolved.

In some examples, the normal temperature in the step (ii) is 20°C-35°C. In some examples, the normal temperature in the step (ii) is 25°C-30°C.

In some examples, the heating to 65°C-75°C in the step (iv) is heating to 65°C, 66°C, 67°C, 68°C, 69°C, 70°C, 71°C, 72°C, 73°C, 74°C and 75°C.

In some examples, the homogenization speed of the homogenization in the step (iv) is 1500 rpm-2500 rpm. In some examples, the homogenization speed of the homogenization in the step (iv) is 1500 rpm, 1600, 1700, 1800, 1900, 2000, 2100, 2200, 2300, 2400 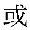 2500 rpm.

In some examples, the time of the homogenization in the step (iv) is 5 minutes-30 minutes. In some examples, the time of the homogenization in the step (iv) is 5 minutes, 10 minutes, 15 minutes, 20 minutes, 25 minutes or 30 minutes.

### Beneficial effects

Compared with the prior art, the technical schemes provided by the present application have at least one beneficial technical effect as follows:
(1) Compared with the formula lacking any component of 3- o-ethyl ascorbic acid, the polyol, the nonionic surfactant and the anionic surfactant, the glabridin composition with the formula provided by the present application is more beneficial to enhancing the permeability of the essence dosage form, thus having unexpected technical effects.
(2) Compared with other preparation methods (for example, the addition sequence of 3-o-ethyl ascorbic acid is different), using the glabridin composition obtained by the preparation process provided by the present application is more beneficial to enhancing the permeability of the essence dosage form, thus having unexpected technical effects.
(3) Compared with cyclodextrin inclusion and liposome inclusion, using the glabridin composition obtained by the preparation process provided by the present application is more beneficial to enhancing the permeability of the essence dosage form, thus having unexpected technical effects.
(4) Compared with other nonionic surfactants, the glabridin composition obtained by using oleth-20 and polyglycerol-10 oleate as nonionic surfactants in the present application is more beneficial to enhancing the permeability of the essence dosage form, thus having unexpected technical effects.
(5) Compared with other polyols, the glabridin composition obtained by using 1,3-butanediol as a polyol in the present application is more beneficial to enhancing the permeability of the essence dosage form, thus having unexpected technical effects.
(6) Compared with other oils, the glabridin composition obtained by using caprylic acid/capric acid triglyceride as an oil in the present application is more beneficial to enhancing the permeability of the essence dosage form, thus having unexpected technical effects.
(7) Compared with not adding 3-o-ethyl ascorbic acid, the glabridin composition with the formula provided by the present application is more beneficial to enhancing the onset rate and effect of the clinical whitening and spot removing efficacy of the essential dosage form, thus having unexpected technical effects.
(8) Compared with other preparation methods (for example, the addition sequence of 3-o-ethyl ascorbic acid is different), using the glabridin composition obtained by the preparation process provided by the present application is more beneficial to enhancing the onset rate and effect of the clinical whitening and spot removing efficacy of the essential dosage form, thus having unexpected technical effects.
(9) Compared with cyclodextrin inclusion and liposome inclusion, using the glabridin composition obtained by the preparation process provided by the present application is more beneficial to enhancing the onset rate and effect of the clinical whitening and spot removing efficacy of the essential dosage form, thus having unexpected technical effects.
(10) Compared with the formula lacking any component of 3- o-ethyl ascorbic acid, the polyol, the nonionic surfactant and the anionic surfactant, using the formula provided by the present application is more beneficial to enhancing the state stability and content stability of the glabridin composition under high temperature and low temperature conditions.
(11) Compared with other nonionic surfactants, using oleth-20 and polyglycerol-10 oleate in the present application is more beneficial to enhancing the state stability and content stability of the glabridin composition under high temperature and low temperature conditions.
(12) Compared with other polyols, using 1,3-butanediol in the present application is more beneficial to enhancing the state stability and content stability of the glabridin composition under high temperature conditions.
(13) Compared with other oils, using caprylic acid/capric acid triglyceride in the present application is more beneficial to enhancing the state stability and content stability of the glabridin composition under high temperature conditions.
(14) Compared with other anionic surfactants, using sodium stearoyl glutamate in the present application is more beneficial to enhancing the state stability and content stability of the glabridin composition under high temperature and low temperature conditions.
(15) Compared with other preparation methods (for example, the addition sequence of 3-o-ethyl ascorbic acid or the addition sequence of water is different), using the preparation process provided by the present application is more beneficial to enhancing the state stability and content stability of the glabridin composition under high temperature and low temperature conditions.
(16) Compared with other preparation methods (for example, the addition sequence of 3-o-ethyl ascorbic acid is different), using the preparation process provided by the present application is more beneficial to enhancing the pH stability of the glabridin composition under high temperature and low temperature conditions.
(17) Compared with not adding 3-o-ethyl ascorbic acid, the formula provided by the present application is more beneficial to enhancing the safety of the glabridin composition, thus having unexpected technical effects.
(18) Compared with other preparation methods (for example, the addition sequence of 3-o-ethyl ascorbic acid is different), using the preparation process provided by the present application is more beneficial to enhancing the safety of the glabridin composition.
(19) The preparation method of the glabridin composition provided by the present application is simple, need simple equipment which is all conventional equipment in the field of cosmetics, and does not need to use common equipment for non-cosmetic production, which is beneficial to reducing the preparation cost.

### Terminology description

In the foregoing description of the present application, all numbers disclosed herein are approximations, whether or not the words "approximately" or "about" are used. Based on the figures disclosed, there may be a difference of less than ±10% in the numerical value of each number or a reasonable difference considered by those in the field, such as a difference of ±1%, ±2%, ±3%, ±4% or ±5%.

The term "normal temperature" means the indoor ambient temperature, which is 20°C-35°C, or 25°C-30°C.

The term "and/or" should be understood to mean any one of the options or a combination of any two or more of the options.

The term "wt%" means mass percentage.

In the description of this specification, descriptions referring to the terms "an example", "some examples", "embodiments", "specific embodiments" or "some embodiments" and so on mean that specific characteristics, structures, materials or features described in connection with this example or embodiment are included in at least one example or embodiment of the present application. In this specification, the schematic expressions of the above terms are not necessarily aimed at the same example or embodiment. Moreover, the specific characteristics, structures, materials or features described may be combined in any one or more examples or embodiments in a suitable manner. In addition, those skilled in the art can associate and combine different examples or embodiments and characteristics of different examples or embodiments described in this specification without contradicting each other.

### DETAILED DESCRIPTION

In order to make those skilled in the art better understand the technical schemes of the present application, some non-limiting examples are further disclosed below to further explain the present application in detail.

The reagents used in the present application can all be commercially available or can be prepared by the method described in the present application.

### I. Test method or detection method of glabridin content

For the test method of glabridin content, please refer to Q/FZR 0101-2023, Raw material for cosmetics-glabridin, wherein the chromatographic conditions are specifically as follows:
a) chromatographic column: ZORBAX StableBond C18, 4.6x250 mm, 5 um;
b) flow rate: 1.0 mL/min;
c) mobile phase: acetonitrile:water =80:20, filtered with a 0.22 pm membrane filter;
d) sample injection volume: 10 uL;
e) column temperature: 25°C;
f) wavelength: 280 nm.

### II. Reagent

Source of aqueous peony flower solution: product name: FangYe5031 Peony Hydrosols, purchased from "Shandong Fangye Biotechnology Co., Ltd.", batch number: 20231231B5031; INCI: aqueous peony (PAEONIA SUFFRUTICOSA) flower solution, p-hydroxyacetophenone, 1,2-hexanediol; wherein, the content of each component is: aqueous peony (PAEONIA SUFFRUTICOSA) flower solution 99%, p-hydroxyacetophenone 0.5% and 1,2-hexanediol 0.5%.

### Example 1-Example 6: Preparation of glabridin composition

Formula: See Table 1.

**Table 1: Formula of glabridin Composition**

| **Split phases** | **Component** | | **Example 1** | **Example 2** | **Exam ple 3** | **Exampl e 4** | **Exampl e 5** | **Example 6** |
|---|---|---|---|---|---|---|---|---|
| S1 phase | oil | caprylic acid/capri c acid triglycerid e | 10.00 wt% | 10.00 wt% | 10.00 wt% | 20.00 wt% | 40.00 wt% | 1.00 wt% |
| | activ e ingre dient | glabridin | 1.00 wt% | 1.00 wt% | 1.00 wt% | 2.00 wt% | 4.00 wt% | 0.10 wt% |
| S2 phase | 3-o-e thyl ascorbic acid | 3-o-ethyl ascorbic acid | 1.00 wt% | 1.00 wt% | 2.00 wt% | 2.00 wt% | 4.00 wt% | 0.10 wt% |
| | poly ol | 1,3-butan ediol | 6.00 wt% | 6.00 wt% | 6.00 wt% | 6.00 wt% | 6.00 wt% | 0.60 wt% |
| | noni onic surfa ctant | oleth-20 | 0.50 wt% | 1.00 wt% | 0.50 wt% | 2.00 wt% | 4.00 wt% | 0.10 wt% |
| | | polyglyce rol-10 oleate | 6.00 wt% | 6.00 wt% | 6.00 wt% | 6.00 wt% | 6.00 wt% | 0.60 wt% |
| | anio nic surfa ctant | sodium stearoyl glutamate | 1.00 wt% | 1.00 wt% | 1.00 wt% | 1.00 wt% | 1.00 wt% | 0.10 wt% |
| | wate r | water | 3.00 wt% | 3.00 wt% | 3.00 wt% | 3.00 wt% | 3.00 wt% | 0.30 wt% |
| S3 phase | wate r | water | balance | balance | balanc e | balance | balance | balance |

### Preparation method:

(1) Glabridin and the oil were mixed, heated to 70 °C for dissolution, and uniformly mixed to obtain the S1 phase;
(2) 3-o-ethyl ascorbic acid, the anionic surfactant, the nonionic surfactant, the polyol and water were mixed, heated to 60 °C for dissolution, and uniformly mixed to obtain the S2 phase;
(3) the S1 phase obtained in the step (1) was added into the S2 phase obtained in the step (2) while the S2 phase was stirred, and mixed, to form a gel; and
(4) then the gel obtained in the step (3) was mixed with the S3 phase to obtain the glabridin composition.

### Example 7- Example 11: Essence dosage form

**Formula: See Table 2.**

**Table 2: Formula of essence dosage form**

| **Split phases** | **Component** | **Example 7** | **Example 8** | **Example 9** | **Example 10** | **Example 11** |
|---|---|---|---|---|---|---|
| A phase | water | balance | balance | balance | balance | balance |
| | EDTA disodium | 0.050 wt% | 0.050 wt% | 0.050 wt% | 0.100 wt% | 0.020 wt% |
| | carbomer | 0.200 wt% | 0.200 wt% | 0.200 wt% | 0.500 wt% | 0.100 wt% |
| B phase | p-hydroxyacetopheno ne | 0.100 wt% | 0.100 wt% | 0.100 wt% | 0.300 wt% | 0.050 wt% |
| | 1,2-pentanediol | 3.000 wt% | 3.000 wt% | 3.000 wt% | 5.000 wt% | 1.000 wt% |
| | 1,2-hexanediol | 0.500 wt% | 0.500 wt% | 0.500 wt% | 1.000 wt% | 0.500 wt% |
| C phase | water | 1.000 wt% | 1.000 wt% | 1.000 wt% | 2.000 wt% | 1.000 wt% |
| | arginine | 0.200 wt% | 0.200 wt% | 0.200 wt% | 0.500 wt% | 0.100 wt% |
| D phase | hydrogenated lecithin | 0.060 wt% | 0.060 wt% | 0.060 wt% | 0.100 wt% | 0.040 wt% |
| | ethoxydiglycol | 0.020 wt% | 0.020 wt% | 0.020 wt% | 0.100 wt% | 0.010 wt% |
| | palmitoyl tripeptide-8 | 0.002 wt% | 0.002 wt% | 0.002 wt% | 0.005 wt% | 0.001 wt% |
| | alpha-tocopherol | 0.005 wt% | 0.005 wt% | 0.005 wt% | 0.010 wt% | 0.005 wt% |
| E phase | glutathione | 0.010 wt% | 0.010 wt% | 0.010 wt% | 0.050 wt% | 0.001 wt% |
| | aqueous peony flower solution | 0.100 wt% | 0.100 wt% | 0.100 wt% | 0.500 wt% | 0.050 wt% |
| F phase | / | glabridin compositio n obtained in Example 1: 1.000 wt% | glabridin compositio n obtained in Example 2: 1.000 wt% | glabridin compositio n obtained in Example 3: 1.000 wt% | glabridin compositio n obtained in Example 1: 2.000 wt% | glabridin compositio n obtained in Example 1: 1.000 wt% |

### Preparation method:

(1) Each component of the A phase was mixed, heated to 80°C for 30 minutes, stirred until they were completely dissolved, and then cooled to 45°C to obtain the A phase;
(2) each component of the B phase was mixed, heated to 65°C, stirred until they were completely dissolved, and then cooled to normal temperature to obtain the B phase;
(3) each component of the C phase was stirred until they were completely dissolved to obtain the C phase;
(4) each component of the D phase was mixed and stirred, heated to 70°C, and homogenized at 2000 rpm for 10 minutes to obtain the D phase; and
(5) the B phase, the C phase, the D phase, the E phase and the F phase were added into the A phase obtained in the step (1) while the A phase was stirred, and mixed, to obtain the cosmetic product with the essence dosage form.

### Comparative Example 1-Comparative Example 5: Glabridin Composition

Formula: See Table 3.

**Table 3: Formulas of glabridin Compositions of Comparative Example 1-Comparative Example 5**

| **Split phases** | **Component** | | **Compara tive Example 1** | **Comparati ve Example 2** | **Comparati ve Example 3** | **Compara tive Example 4** | **Compara tive Example 5** |
|---|---|---|---|---|---|---|---|
| S1 phase | oil | caprylic acid/capri c acid triglycerid e | 10.00 wt% | 10.00 wt% | 10.00 wt% | 10.00 wt% | 10.00 wt% |
| | acti ve ingr edie nt | glabridin | 1.00 wt% | 1.00 wt% | 1.00 wt% | 1.00 wt% | 1.00 wt% |
| S2 phase | 3-o-eth yl asc orbi c acid | 3-o-ethyl ascorbic acid | 0 | 1.00 wt% | 1.00 wt% | 1.00 wt% | 1.00 wt% |
| | polyol | 1,3-butanediol | 6.00 wt% | 6.00 wt% | 6.00 wt% | 6.00 wt% | 0 |
| | non ioni c surf acta nt | oleth-20 | 0.50 wt% | 0 | 6.50 wt% | 0.50 wt% | 0.50 wt% |
| | | polyglyce rol-10 oleate | 6.00 wt% | 6.50 wt% | 0 | 7.00 wt% | 6.00 wt% |
| | ani oni c surf acta nt | sodium stearoyl glutamate | 1.00 wt% | 1.00 wt% | 1.00 wt% | 0 | 1.00 wt% |
| | wat er | water | 3.00 wt% | 3.00 wt% | 3.00 wt% | 3.00 wt% | 3.00 wt% |
| S3 phase | wat er | water | balance | balance | balance | balance | balance |

Preparation method: The preparation was carried out according to the preparation method of Example 1, except that the component with the content of 0 was not added, and each of other components was added according to the content of the corresponding component in each comparative example.

### Comparative Example 6-Comparative Example 11: Investigation of nonionic surfactant

Formula: See Table 4.

**Table 4: Investigation formula of nonionic surfactant**

| **Split phases** | **Component** | | **Compa rative Exampl e 6** | **Compa rative Exampl e 7** | **Compa rative Exampl e 8** | **Compa rative Exampl e 9** | **Compa rative Exampl e 10** | **Compa rative Exampl e 11** |
|---|---|---|---|---|---|---|---|---|
| S1 phase | oil | caprylic acid/capric acid triglyceride | 10.00 wt% | 10.00 wt% | 10.00 wt% | 10.00 wt% | 10.00 wt% | 10.00 wt% |
| | active ingredi ent | glabridin | 1.00 wt% | 1.00 wt% | 1.00 wt% | 1.00 wt% | 1.00 wt% | 1.00 wt% |
| S2 phase | 3-o-eth yl ascorbi c acid | 3-o-ethyl ascorbic acid | 1.00 wt% | 1.00 wt% | 1.00 wt% | 1.00 wt% | 1.00 wt% | 1.00 wt% |
| | polyol | 1,3-butaned iol | 6.00 wt% | 6.00 wt% | 6.00 wt% | 6.00 wt% | 6.00 wt% | 6.00 wt% |
| | nonion ic surfact ant | oleth-20 | 0 | 0 | 0 | 0.50 wt% | 0.50 wt% | 0.50 wt% |
| | | polyglycero 1-10 oleate | 6.00 wt% | 6.00 wt% | 6.00 wt% | 0 | 0 | 0 |
| | | polysorbate -80 | 0.50 wt% | 0 | 0 | 0 | 0 | 0 |
| | | sorbitan oleate | 0 | 0.50 wt% | 0 | 0 | 0 | 0 |
| | | mixture of polysorbate -80 and sorbitan oleate (mass ratio: 10:3) | 0 | | 0.50 wt% | 0 | 0 | 0 |
| | | polyglycero 1-10 stearate | 0 | 0 | 0 | 6.00 wt% | 0 | 0 |
| | | polyglycero 1-10 myristate | 0 | 0 | 0 | 0 | 6.00 wt% | 0 |
| | | polyglycero 1-10 dioleate | 0 | 0 | 0 | 0 | 0 | 6.00 wt% |
| | anioni c surfact ant | sodium stearoyl glutamate | 1.00 wt% | 1.00 wt% | 1.00 wt% | 1.00 wt% | 1.00 wt% | 1.00 wt% |
| | water | water | 3.00 wt% | 3.00 wt% | 3.00 wt% | 3.00 wt% | 3.00 wt% | 3.00 wt% |
| S3 phase | water | water | balance | balance | balance | balance | balance | balance |

Preparation method: The preparation was carried out according to the preparation method of Example 1, except that the component with the content of 0 was not added, and each of other components was selected according to the corresponding component in each comparative example and added according to the content of the corresponding component.

### Comparative Example 12-Comparative Example 14: Investigation of anionic surfactant

Formula: See Table 5.

**Table 5: Investigation formula of anionic surfactant**

| **Split phases** | **Component** | | **Comparative Example 12** | **Comparative Example 13** | **Comparative Example 14** |
|---|---|---|---|---|---|
| S1 phase | oil | caprylic acid/capric acid triglyceride | 10.00 wt% | 10.00 wt% | 10.00 wt% |
| | active ingredient | glabridin | 1.00 wt% | 1.00 wt% | 1.00 wt% |
| S2 phase | 3-o-ethyl ascorbic | 3-o-ethyl ascorbic acid | 1.00 wt% | 1.00 wt% | 1.00 wt% |
| | acid | | | | |
| | polyol | 1,3-butanediol | 6.00 wt% | 6.00 wt% | 6.00 wt% |
| | nonionic surfactant | oleth-20 | 0.50 wt% | 0.50 wt% | 0.50 wt% |
| | | polyglycerol-1 0 oleate | 6.00 wt% | 6.00 wt% | 6.00 wt% |
| | **anionic surfactant** | potassium cetyl phosphate | 1.00 wt% | 0 | 0 |
| | | sodium di(lauramidog lutamide)lysin e | 0 | 1.00 wt% | 0 |
| | | tri(laureth-4)p hosphate | 0 | 0 | 1.00 wt% |
| | water | water | 3.00 wt% | 3.00 wt% | 3.00 wt% |
| S3 phase | water | water | balance | balance | balance |

Preparation method: The preparation was carried out according to the preparation method of Example 1, except that different anionic surfactants were used.

### Comparative Example 15-Comparative Example 18: Investigation of oil

Formula: See Table 6.

**Table 6: Investigation formula of oil**

| **Split phases** | **Component** | | **Comparative Example 15** | **Comparative Example 16** | **Comparative Example 17** | **Comparative Example 18** |
|---|---|---|---|---|---|---|
| S1 phase | oil | olive oil | 10.00 wt% | 0 | 0 | 0 |
| | | castor oil | 0 | 10.00 wt% | 0 | 0 |
| | | hydroge nated polydec ene | 0 | 0 | 10.00 wt% | 0 |
| | | phytoste rol ester | 0 | 0 | 0 | 10.00 wt% |
| S2 phase | active ingredi ent | glabridi n | 1.00 wt% | 1.00 wt% | 1.00 wt% | 1.00 wt% |
| | 3-o-eth yl ascorbi c acid | 3-o-ethy 1 ascorbic acid | 1.00 wt% | 1.00 wt% | 1.00 wt% | 1.00 wt% |
| | polyol | 1,3-buta nediol | 6.00 wt% | 6.00 wt% | 6.00 wt% | 6.00 wt% |
| | nonionic surfact ant | oleth-20 | 0.50 wt% | 0.50 wt% | 0.50 wt% | 0.50 wt% |
| | | polyglyc erol-10 oleate | 6.00 wt% | 6.00 wt% | 6.00 wt% | 6.00 wt% |
| | anionic surfact ant | sodium stearoyl glutamat e | 1.00 wt% | 1.00 wt% | 1.00 wt% | 1.00 wt% |
| | water | water | 3.00 wt% | 3.00 wt% | 3.00 wt% | 3.00 wt% |
| S3 phase | water | water | balance | balance | balance | balance |

Preparation method: The preparation was carried out according to the preparation method of Example 1, except that different oils were used.

### Comparative Example 19-Comparative Example 21: Investigation of polyol

Formula: See Table 7.

**Table 7: Investigation formula of polyol**

| **Split phases** | **Component** | | **Comparative Example 19** | **Comparative Example 20** | **Comparative Example 21** |
|---|---|---|---|---|---|
| S1 phase | oil | caprylic acid/capric acid triglyceride | 10.00 wt% | 10.00 wt% | 10.00 wt% |
| | active ingredient | glabridin | 1.00 wt% | 1.00 wt% | 1.00 wt% |
| S2 phase | 3-o-ethyl ascorbic acid | 3-o-ethyl ascorbic acid | 1.00 wt% | 1.00 wt% | 1.00 wt% |
| | polyol | glycerol | 6.00 wt% | 0 | 0 |
| | | sorbitol | 0 | 6.00 wt% | 0 |
| | | 1,3-propanediol | 0 | 0 | 6.00 wt% |
| | nonionic surfactant | oleth-20 | 0.50 wt% | 0.50 wt% | 0.50 wt% |
| | | polyglycerol-10 oleate | 6.00 wt% | 6.00 wt% | 6.00 wt% |
| | anionic surfactant | sodium stearoyl glutamate | 1.00 wt% | 1.00 wt% | 1.00 wt% |
| | water | water | 3.00 wt% | 3.00 wt% | 3.00 wt% |
| S3 phase | water | water | balance | balance | balance |

Preparation method: The preparation was carried out according to the preparation method of Example 1, except that different polyols were used.

### Comparative Example 22: Investigation of preparation method

The formula of this comparative example is shown in Table 8.

**Table 8: Formula of Comparative Example 22**

| **Split phases** | **Component** | | **Comparative Example 22** |
|---|---|---|---|
| S1 phase | oil | caprylic acid/capric acid triglyceride | 10.00 wt% |
| | active ingredient | glabridin | 1.00 wt% |
| S2 phase | polyol | 1,3-butanediol | 6.00 wt% |
| | nonionic surfactant | oleth-20 | 0.50 wt% |
| | | polyglycerol-10 oleate | 6.00 wt% |
| | anionic surfactant | sodium stearoyl glutamate | 1.00 wt% |
| | water | water | 3.00 wt% |
| S3 phase | 3-o-ethyl ascorbic acid | 3-o-ethyl ascorbic acid | 1.00 wt% |
| | water | water | balance |

### Preparation method:

Compared with Example 1, this comparative example was different in that 3-o-ethyl ascorbic acid in the S1 phase was added in the step (4) in the preparation method, and the detailed steps were as follows:
(1) glabridin and the oil were mixed, heated to 70 °C for dissolution, and uniformly mixed to obtain the S1 phase;
(2) the nonionic surfactant, the anionic surfactant, the polyol and water were mixed, heated to 60 °C for dissolution, and uniformly mixed to obtain the S2 phase;
(3) the S1 phase obtained in the step (1) was added into the S2 phase obtained in the step (2) while the S2 phase was stirred, and mixed, to form a gel; and
(4) then the gel obtained in the step (3) was mixed with water in the S3 phase and 3-o-ethyl ascorbic acid to obtain the glabridin composition.

### Comparative Example 23: Investigation of preparation method

The formula of this comparative example is shown in Table 9.

**Table 9: Formula of Comparative Example 23**

| **Split phases** | **Component** | | **Comparative Example 23** |
|---|---|---|---|
| S1 phase | oil | caprylic acid/capric acid triglyceride | 10.00 wt% |
| | active ingredient | glabridin | 1.00 wt% |
| S2 phase | polyol | 1,3-butanediol | 6.00 wt% |
| | nonionic surfactant | oleth-20 | 0.50 wt% |
| | | polyglycerol-10 oleate | 6.00 wt% |
| | anionic surfactant | sodium stearoyl glutamate | 1.00 wt% |
| | water | water | balance |
| S3 phase | 3-o-ethyl ascorbic acid | 3-o-ethyl ascorbic acid | 1.00 wt% |

### Preparation method:

Compared with the preparation method of Example 1, the preparation method of this comparative example was different in the feeding sequence of components in the preparation method, and the specific steps were as follows:
(1) glabridin and the oil were mixed, heated to 70 °C for dissolution, and uniformly mixed to obtain the S1 phase;
(2) the nonionic surfactant, the anionic surfactant, the polyol and water were mixed, heated to 60 °C for dissolution, and uniformly mixed to obtain the S2 phase;
(3) the S1 phase obtained in the step (1) was added into the S2 phase obtained in the step (2) while the S2 phase was stirred, and mixed, to obtain a mixture (note: no gel would be formed here); and
(4) then the mixture obtained in the step (3) was mixed with 3-o-ethyl ascorbic acid to obtain the glabridin composition.

### Comparative Example 24: Investigation of cyclodextrin dosage form

**Table 10: Formula of Comparative Example 24**

| **Split phases** | **Component** | **Addition amount** |
|---|---|---|
| S1 phase | 2- sulfobutyl-β-cyclodextrin | 4.50 wt% |
| | water | 64.50 wt% |
| S2 phase | glabridin | 1.00 wt% |
| | ethanol | 30.00 wt% |

### Preparation method:

The S1 phase was stirred until it was completely dissolved at normal temperature, and the S2 phase was stirred until it was completely dissolved at normal temperature; the S1 and S2 phases were mixed and shaken for 24 h at 200 r/min to obtain a clear and transparent solution; then, the solution was treated with a rotary evaporator at 45°C to remove ethanol, and was further frozen in a refrigerator at -20°C for 12 h, and then freeze-dried in a freeze dryer to prepare the glabridin composition (cyclodextrin dosage form) of Comparative Example 24.

### Comparative Example 25: Investigation of liposome dosage form

**Table 11: Formula of Comparative Example 25**

| **Split phases** | **Component** | **Addition amount** |
|---|---|---|
| S1 phase | soybean phospholipid | 3.00 wt% |
| | palmityl palmitate | 1.00 wt% |
| | isopropyl myristate | 2.00 wt% |
| | octyldodecanol | 2.00 wt% |
| | glabridin | 2.00 wt% |
| S2 phase | vitamin E polyethylene glycol succinate | 1.00 wt% |
| | 1,3-butanediol | 10.00 wt% |
| | 1,3-propanediol | 5.00 wt% |
| | water | 74.00 wt% |

### Preparation method:

The S1 phase was heated to 80°C and stirred until it was completely dissolved, and the S2 phase was heated to 80°C and stirred until it was completely dissolved; the S1 phase was added dropwise into the S2 phase, stirred continuously, sheared and emulsified at high speed of 10000 rpm for 1 min to prepare a micron dispersion; the micron dispersion was homogenized at high pressure and circulated for 5 times at 1500 bar to prepare the glabridin composition (in the dosage form of liposome) of Comparative Example 25.

### Example 26- Example 50: Essence dosage form

Comparative Examples 1-25 were added to the formula of the essence dosage form, and the formula was shown in Table 12.

**Table 12: Adding Comparative Examples 1-25 to formula of essence dosage form**

| **Split phases** | **Addition amount** | **Essence formulas of Comparative Example 26-Comparative Example 49** | **Essence formula of Comparative Example 50** |
|---|---|---|---|
| A phase | water | balance | balance |
| | EDTA disodium | 0.050 wt% | 0.050 wt% |
| | carbomer | 0.200 wt% | 0.200 wt% |
| B phase | p-hydroxyacetophe none | 0.100 wt% | 0.100 wt% |
| | 1,2-pentanediol | 3.000 wt% | 3.000 wt% |
| | 1,2-hexanediol | 0.500 wt% | 0.500 wt% |
| C phase | water | 1.000 wt% | 1.000 wt% |
| | arginine | 0.200 wt% | 0.200 wt% |
| D phase | hydrogenated lecithin | 0.060 wt% | 0.060 wt% |
| | ethoxydiglycol | 0.020 wt% | 0.020 wt% |
| | palmitoyl tripeptide-8 | 0.002 wt% | 0.002 wt% |
| | alpha-tocopherol | 0.005 wt% | 0.005 wt% |
| E phase | glutathione | 0.010 wt% | 0.010 wt% |
| | aqueous peony flower solution | 0.100 wt% | 0.100 wt% |
| F phase | / | glabridin compositions of Comparative Example 1-Comparative Example 24 (the glabridin compositions of Comparative Example 1-Comparative Example 24 were used correspondingly in turn in Comparative Example 26-Comparative Example): 1.000 wt% | glabridin composition obtained in Comparative Example 25: 0.500 wt% |

Preparation method: The preparation was carried out according to the preparation method of Examples 7-11, except that the F phase was replaced by the glabridin composition obtained in Comparative Example 1- Comparative Example 25, respectively, and the composition was added according to the component contents in Table 12.

### Experimental Example 1: Permeability experiment

### Test method:

(1) According to "GB-T27818-2011, Chemicals-testing method for skin absorption-in vitro" and "Q/FZR 0101-2023, Raw material for cosmetics-glabridin", the essence dosage forms of Example 7-Example 9 and Comparative Example 26, Comparative Example 47, Comparative Example 49 and Comparative Example 50 were used respectively to study the permeation behavior of the samples at 2 h, 4 h, 8 h, 12 h and 24 h based on the piglet skin-Franz cell system, as shown in Table 13.
(2) The essence dosage forms obtained in Comparative Example 27-Comparative Example 46 and Comparative Example 48 were used respectively to study the permeation behavior of the samples at 24 h based on the piglet skin-Franz cell system, as shown in Table 14.

In this experiment, three parallel groups were set up to obtain the average value. The test results are shown in Table 13 and Table 14.

**Table 13: Investigation results of permeability at different Time/ (ug/cm²)**

| Tim e point | Permeability (ug/cm²) | | | | | | | | |
|---|---|---|---|---|---|---|---|---|---|
| | Example 7 | Exampl e 8 | Exampl e 9 | Example 10 | Exampl e 11 | Compar ative Exampl e 26 | Compar ative Exampl e 47 | Compar ative Exampl e 49 | Compa rative Examp le 50 |
| 2h | 25.08 | 28.11 | 30.05 | 50.34 | 26.25 | 12.65 | 15.45 | 10.76 | 18.96 |
| 4h | 35.17 | 39.43 | 40.90 | 70.07 | 38.66 | 17.43 | 20.05 | 16.85 | 29.69 |
| 8h | 51.81 | 55.55 | 62.25 | 99.31 | 52.30 | 24.78 | 28.64 | 20.68 | 35.78 |
| 12 h | 64.71 | 67.56 | 74.75 | 122.48 | 62.37 | 31.30 | 36.38 | 25.88 | 46.65 |
| 24 h | 85.22 | 95.69 | 104.87 | 170.55 | 92.85 | 45.12 | 53.79 | 37.73 | 62.37 |

**Table 14: Investigation results of permeability at 24 h (ug/cm²)**

| **Sample** | Comparative Example 27 | Comparat ive Example 28 | Compar ative Exampl e 29 | Compar ative Exampl e 30 | Compa rative Exampl e 31 | Compar ative Exampl e 32 | Compar ative Exampl e 33 |
|---|---|---|---|---|---|---|---|
| **permeability at 24 h (ug/cm²)** | 8.25 | 7.25 | 6.87 | 15.34 | 6.35 | 7.49 | 9.52 |
| **Sample** | Comparative Example 34 | Comparat ive Example 35 | Compar ative Exampl e 36 | Compar ative Exampl e 37 | Compa rative Exampl e 38 | Compar ative Exampl e 39 | Compar ative Exampl e 40 |
| **permeability at 24 h (ug/cm²)** | 28.67 | 35.53 | 40.74 | 45.90 | 46.90 | 48.79 | 48.55 |
| **Sample** | Comparative Example 41 | Comparat ive Example 42 | Compar ative Exampl e 43 | Compar ative Exampl e 44 | Compa rative Exampl e 45 | Compar ative Exampl e 46 | Compar ative Exampl e 48 |
| **permeability at 24 h (ug/cm²)** | 36.63 | 45.54 | 7.49 | 32.56 | 35,21 | 38.59 | 2.08 |

### Conclusion:

(1) Compared with the formula lacking any component of 3- o-ethyl ascorbic acid, the polyol, the nonionic surfactant and the anionic surfactant, the glabridin composition with the formula provided by the present application is more beneficial to enhancing the permeability of the essence dosage form, thus having unexpected technical.
(2) Compared with other preparation methods (for example, the addition sequence of 3-o-ethyl ascorbic acid is different), using the glabridin composition obtained by the preparation process provided by the present application is more beneficial to enhancing the permeability of the essence dosage form, thus having unexpected technical effects.
(3) Compared with cyclodextrin inclusion and liposome inclusion, using the glabridin composition obtained by the preparation process provided by the present application is more beneficial to enhancing the permeability of the essence dosage form, thus having unexpected technical effects.
(4) Compared with other nonionic surfactants, the glabridin composition obtained by using oleth-20 and polyglycerol-10 oleate as nonionic surfactants in the present application is more beneficial to enhancing the permeability of the essence dosage form, thus having unexpected technical effects.
(5) Compared with other polyols, the glabridin composition obtained by using 1,3-butanediol as a polyol in the present application is more beneficial to enhancing the permeability of the essence dosage form, thus having unexpected technical effects.
(6) Compared with other oils, the glabridin composition obtained by using caprylic acid/capric acid triglyceride as an oil in the present application is more beneficial to enhancing the permeability of the essence dosage form, thus having unexpected technical effects.

### Experimental Example 2: Verification of clinical whitening efficacy

Test method: According to First method-test methods for whitening efficacy of human skin blackening model induced by ultraviolet rays, the essence dosage forms of Example 7-Example 9 and Comparative Example 26, Comparative Example 47, Comparative Example 49 and Comparative Example 50 were used respectively to see whether the difference of the skin color visual scores, the ITA° difference or the MI difference at any time point before and after application of the test product was significantly improved compared with the negative control (P<0.05), otherwise, it was considered that the test product has no spot removing and whitening efficacy. The test results are shown in Table 15.

**Table 15: Results of differences before and after application of test products compared with negative control**

| Parameter | After use | Examp le 7 | Exam ple 8 | Exam ple 9 | Exam ple 10 | Exam ple 11 | Comp arativ e Exam ple 26 | Comp arativ e Exam ple 47 | Comp arativ e Exam ple 48 | Comp arativ e Exam ple 50 |
|---|---|---|---|---|---|---|---|---|---|---|
| ΔITA° | 1 week | 0.186 | 0.252 | 0.238 | 0.256 | 0.287 | 0.245 | 0.316 | 0.252 | 0.276 |
| | 2 week | 0.012 | 0.046 | 0.026 | 0.005 | 0.041 | 0.258 | 0.250 | 0.273 | 0.248 |
| | 3 week | 0.008 | 0.012 | 0.013 | 0.002 | 0.010 | 0.165 | 0.157 | 0.184 | 0.110 |
| | 4 week | 0.002 | 0.006 | 0.005 | 0.002 | 0.005 | 0.038 | 0.018 | 0.071 | 0.044 |
| ΔMI | 1 week | 0.249 | 0.354 | 0.295 | 0.346 | 0.326 | 0.325 | 0.368 | 0.317 | 0.298 |
| | 2 week | 0.215 | 0.268 | 0.249 | 0.195 | 0.246 | 0.287 | 0.265 | 0.275 | 0.286 |
| | 3 week | 0.036 | 0.166 | 0.133 | 0.024 | 0.153 | 0.225 | 0.180 | 0.197 | 0.198 |
| | 4 week | 0.012 | 0.035 | 0.032 | 0.009 | 0.021 | 0.065 | 0.051 | 0.096 | 0.049 |
| Δ skin color visual score | 1 week | 0.354 | 0.345 | 0.389 | 0.368 | 0.340 | 0.369 | 0.380 | 0.360 | 0.365 |
| | 2 week | 0.222 | 0.233 | 0.297 | 0.196 | 0.254 | 0.298 | 0.305 | 0.275 | 0.287 |
| | 3 week | 0.168 | 0.126 | 0.154 | 0.102 | 0.131 | 0.224 | 0.198 | 0.168 | 0.175 |
| | 4 week | 0.041 | 0.037 | 0.047 | 0.011 | 0.045 | 0.105 | 0.080 | 0.102 | 0.050 |

### Conclusion:

(1) Compared with not adding 3-o-ethyl ascorbic acid, the glabridin composition with the formula provided by the present application is more beneficial to enhancing the onset rate and effect of the clinical whitening and spot removing efficacy of the essential dosage form, thus having unexpected technical effects.
(2) Compared with other preparation methods (for example, the addition sequence of 3-o-ethyl ascorbic acid is different), using the glabridin composition obtained by the preparation process provided by the present application is more beneficial to enhancing the onset rate and effect of the clinical whitening and spot removing efficacy of the essential dosage form, thus having unexpected technical effects.
(3) Compared with cyclodextrin inclusion and liposome inclusion, using the glabridin composition obtained by the preparation process provided by the present application is more beneficial to enhancing the onset rate and effect of the clinical whitening and spot removing efficacy of the essential dosage form, thus having unexpected technical effects.

### Experimental Example 3: Investigation of state stability and content stability

Test method: According to ISO/TR 18811-2018, Cosmetics-Guidelines on the stability testing of cosmetic products and Q/FZR 0101-2023, Raw material for cosmetics-glabridin, the glabridin compositions prepared in the above examples and comparative examples were respectively placed at 45±2°C and -15±2°C to observe the state stability for 6 months and test the glabridin content after 6 months (the content here represented the ratio of the measured concentration of glabridin in the product to the theoretical concentration multiplied by 100%) (the sampling and detecting time points include 0 day, 7 days, 14 days, 1 month, 3 months and 6 months).The results are shown in Table 16, Table 17 and Table 18. (Note: 1. Stop observing immediately when there is abnormal stability performance of crystal precipitation or delamination, and "/" means that the stability is not observed; 2. The content of samples with crystal precipitation observed by naked eyes will be no longer tested)

**Table 16: State stability at 45±2°C for 6 months**

| Grou p | 0 days | 7 days | 14 days | 1 month | 3 months | 6 months |
|---|---|---|---|---|---|---|
| Exa mple 1 | no precipita tion and no delamin ation | no discoloration, no precipitation and no delamination | no discoloration, no precipitation and no delamination | no discoloration, no precipitation and no delamination | no discoloration, no precipitation and no delamination | slight discoloration, no precipitation and no delamination |
| Exa mple 2 | no precipita tion and no delamin ation | no discoloration, no precipitation and no delamination | no discoloration, no precipitation and no delamination | no discoloration, no precipitation and no delamination | no discoloration, no precipitation and no delamination | slight discoloration, no precipitation and no delamination |
| Exa mple 3 | no precipita tion and no delamin ation | no discoloration, no precipitation and no delamination | no discoloration, no precipitation and no delamination | no discoloration, no precipitation and no delamination | no discoloration, no precipitation and no delamination | slight discoloration, no precipitation and no delamination |
| Exa mple 4 | no precipitation and no delamin ation | no discoloration, no precipitation and no delamination | no discoloration, no precipitation and no delamination | no discoloration, no precipitation and no delamination | no discoloration, no precipitation and no delamination | slight discoloration, no precipitation and no delamination |
| Exa mple 5 | no precipita tion and no delamin ation | no discoloration, no precipitation and no delamination | no discoloration, no precipitation and no delamination | no discoloration, no precipitation and no delamination | no discoloration, no precipitation and no delamination | slight discoloration, no precipitation and no delamination |
| Exa mple 6 | no precipita tion and no delamin ation | no discoloration, no precipitation and no delamination | no discoloration, no precipitation and no delamination | no discoloration, no precipitation and no delamination | no discoloration, no precipitation and no delamination | slight discoloration, no precipitation and no delamination |
| Com parat ive Exa mple 1 | no precipita tion and no delamin ation | no discoloration, no precipitation and no delamination | slight discoloration, no precipitation and no delamination | slight discoloration, no precipitation and no delamination | obvious discoloration, no precipitation and no delamination | obvious discoloration, no precipitation and no delamination |
| Com parat ive Exa mple 2 | crystal precipita tion and no delamin ation | / | / | / | / | / |
| Com parat ive Exa mple 3 | crystal precipita tion and delamin ation | / | / | / | / | / |
| Com parat ive Exa mple 4 | crystal precipita tion and delamin ation | / | / | / | / | / |
| Com parat ive Exa mple 5 | no precipita tion and no delamination | no discoloration, no precipitation and slight delamination | / | / | / | / |
| Com parat ive Exa mple 6 | crystal precipita tion and no delamin ation | / | / | / | / | / |
| Com parat ive Exa mple 7 | crystal precipita tion and no delamin ation | / | / | / | / | / |
| Com parat ive Exa mple 8 | crystal precipita tion and no delamin ation | / | / | / | / | / |
| Com parat ive Exa mple 9 | no precipita tion and no delamin ation | no discoloration, no precipitation and no delamination | no discoloration, no precipitation and no delamination | no discoloration, crystal precipitation and slight delamination | / | / |
| Com parat ive Exa mple 10 | no precipita tion and no delamin ation | no discoloration, no precipitation and no delamination | no discoloration, no precipitation and slight delamination | / | / | / |
| Com parat ive Exa mple 11 | no precipita tion and no delamin ation | no discoloration, no precipitation and no delamination | no discoloration, no precipitation and no delamination | no discoloration, no precipitation and no delamination | slight discoloration, no precipitation and slight delamination | / |
| Com parat ive Exa mple 12 | no precipita tion and no delamin ation | no discoloration, no precipitation and no delamination | no discoloration, no precipitation and no delamination | no discoloration, no precipitation and no delamination | no discoloration, no precipitation and no delamination | no discoloration, crystal precipitation and slight delamination |
| Com parative Exa mple 13 | no precipitation and no delamin ation | no discoloration,no precipitation and no delamination | no discoloration, no precipitation and no delamination | no discoloration, crystal precipitation and slight delamination | / | / |
| Com parat ive Exa mple 14 | no precipita tion and no delamin ation | crystal precipitation and slight delamination | / | / | / | / |
| Com parat ive Exa mple 15 | no precipita tion and no delamin ation | slight discoloration, no precipitation and no delamination | obvious discoloration, no precipitation and no delamination | severe discoloration and taste change, no precipitation and no delamination | severe discoloration and taste change, no precipitation and no delamination | severe discoloration, slight precipitation and no delamination |
| Com parat ive Exa mple 16 | no precipita tion and no delamin ation | slight crystal precipitation and no delamination | / | / | / | / |
| Com parat ive Exa mple 17 | no precipita tion and no delamin ation | no precipitation and no delamination | no precipitation and no delamination | no precipitation and no delamination | no precipitation and no delamination | no precipitation and slight delamination |
| Com parat ive Exa mple 18 | crystal precipita tion and no delamin ation | / | / | / | / | / |
| Com parat ive Exa mple 19 | no precipita tion and no delamin ation | no precipitation and slight delamination | / | / | / | / |
| Com parat ive Exa mple 20 | no precipita tion and no delamin ation | no precipitation and no delamination | no precipitation and slight delamination | / | / | / |
| Com parat ive Exa mple 21 | no precipita tion and no delamin ation | no precipitation and no delamination | / | / | / | / |
| Com parat ive Exa mple 22 | no precipita tion and no delamin ation | no precipitation and no delamination | no discoloration, no precipitation and no delamination | no discoloration, no precipitation and no delamination | slight discoloration, no precipitation and no delamination | slight discoloration, no precipitation and no delamination |
| Com parat ive Exa mple 23 | precipita tion and delamin ation | / | / | / | / | / |
| Com parat ive Exa mple 24 | no precipita tion and no delamin ation | no discoloration, no precipitation and no delamination | slight discoloration, no precipitation and no delamination | slight discoloration, no precipitation and no delamination | obvious discoloration, no precipitation and no delamination | severe discoloration, no precipitation and no delamination |
| Com parat ive Exa mple 25 | no precipita tion and no delamin ation | no discoloration, no precipitation and no delamination | no discoloration, no precipitation and no delamination | slight discoloration, no precipitation and no delamination | slight discoloration, no precipitation and no delamination | obvious discoloration, no precipitation and no delamination |

**Table 17: State stability at -15±2°C for 6 months**

| Group | 0 days | 7 days | 14 days | 1 month | 3 months | 6 months |
|---|---|---|---|---|---|---|
| Example 1 | no precipitation and no delamination | no discoloration, no precipitation and no delamination | no discoloration, no precipitation and no delamination | no discoloration, no precipitation and no delamination | no discoloration, no precipitation and no delamination | no discoloration, no precipitation and no delamination |
| Example 2 | no precipitation and no delamination | no discoloration, no precipitation and no delamination | no discoloration, no precipitation and no delamination | no discoloration, no precipitation and no delamination | no discoloration, no precipitation and no delamination | no discoloration, no precipitation and no delamination |
| Example 3 | no precipitation and no delamination | no discoloration, no precipitation and no delamination | no discoloration, no precipitation and no delamination | no discoloration, no precipitation and no delamination | no discoloration, no precipitation and no delamination | no discoloration, no precipitation and no delamination |
| Example 4 | no precipitation and no delamination | no discoloration, no precipitation and no delamination | no discoloration, no precipitation and no delamination | no discoloration, no precipitation and no delamination | no discoloration, no precipitation and no delamination | no discoloration, no precipitation and no delamination |
| Example 5 | no precipitation and no delamination | no discoloration, no precipitation and no delamination | no discoloration, no precipitation and no delamination | no discoloration, no precipitation and no delamination | no discoloration, no precipitation and no delamination | no discoloration, no precipitation and no delamination |
| Example 6 | no precipitation and no delamination | no discoloration, no precipitation and no delamination | no discoloration, no precipitation and no delamination | no discoloration, no precipitation and no delamination | no discoloration, no precipitation and no delamination | no discoloration, no precipitation and no delamination |
| Comparative Example 1 | no precipitation and no delamination | no discoloration, no precipitation and no delamination | no discoloration, no precipitation and no delamination | no discoloration, no precipitation and no delamination | no discoloration, no precipitation and no delamination | no discoloration, no precipitation and no delamination |
| Comparative Example 2 | crystal precipitation and no delamination | / | / | / | / | / |
| Comparative Example 3 | crystal precipitation and delamination | / | / | / | / | / |
| Comparative Example 4 | crystal precipitation and delamination | / | / | / | / | / |
| Comparative Example 5 | no precipitation and no delamination | crystal precipitation and delamination | / | / | / | / |
| Comparative Example 6 | crystal precipitation and no delamination | / | / | / | / | / |
| Comparative Example 7 | crystal precipitation and no delamination | / | / | / | / | / |
| Comparative Example 8 | crystal precipitation and no delamination | / | / | / | / | / |
| Comparative Example 9 | no precipitation and no delamination | no discoloration, no precipitation and no delamination | no discoloration, no precipitation and no delamination | crystal precipitation and no delamination | / | / |
| Comparative Example 10 | no precipitation and no delamination | no discoloration, no precipitation and no delamination | no discoloration, crystal precipitation and no delamination | / | / | / |
| Comparative Example 11 | no precipitation and no delamination | no discoloration, no precipitation and no delamination | no discoloration, no precipitation and no delamination | no discoloration, no precipitation and no delamination | slight discoloration and no precipitation | / |
| Comparative Example 12 | no precipitation and no delamination | no discoloration, no precipitation and no delamination | no discoloration, no precipitation and no delamination | no discoloration, no precipitation and no delamination | no discoloration, no precipitation and no delamination | no discoloration and slight crystal precipitation |
| Comparative Example 13 | no precipitation and no delamination | no discoloration, no precipitation and no delamination | no discoloration, no precipitation and no delamination | no discoloration, no precipitation and no delamination | / | / |
| Comparative Example 14 | no precipitation and no delamination | crystal precipitation and no delamination | / | / | / | / |
| Comparative Examplea 15 | no precipitation and no delamination | no discoloration, no precipitation and no delamination | no discoloration, no precipitation and no delamination | no discoloration and taste change, no precipitation and no delamination | slight discoloration and taste change, no precipitation and no delamination | slight discoloration, slight precipitation and no delamination |
| Comparative Example 16 | no precipitation and no delamination | slight precipitation and no delamination | / | / | / | / |
| Comparative Example 17 | no precipitation and no delamination | no discoloration, no precipitation and no delamination | no discoloration, no precipitation and no delamination | no discoloration, no precipitation and no delamination | no discoloration, no precipitation and no delamination | no discoloration, obvious crystal precipitation and slight delamination |
| Com parative Example 18 | crystal precipitation and no delamination | / | / | / | / | / |
| Comparative Example 19 | no precipitation and no delamination | no discoloration, no precipitation and no delamination | / | / | / | / |
| Comparative Example 20 | no precipitation and no delamination | no discoloration, no precipitation and no delamination | no discoloration, no precipitation and no delamination | / | / | / |
| Comparative Example 21 | no precipitation and no delamination | slight crystal precipitation and no delamination | / | / | / | / |
| Comparative Example 22 | no precipitation and no delamination | no discoloration, no precipitation and no delamination | no discoloration, no precipitation and no delamination | no discoloration, no precipitation and no delamination | no discoloration, no precipitation and no delamination | no discoloration, no precipitation and no delamination |
| Comparative Example 23 | precipitation and delamination | / | / | / | / | / |
| Comparative Example 24 | no precipitation and no delamination | no discoloration, no precipitation and no delamination | no discoloration, no precipitation and no delamination | no discoloration, no precipitation and no delamination | no discoloration, no precipitation and no delamination | slight discoloration, no precipitation and no delamination |
| Comparative Example 25 | no precipitation and no delamination | no precipitation and no delamination | no precipitation and no delamination | no precipitation and no delamination | no precipitation and no delamination | no precipitation and no delamination |

**Table 18: Content stability**

| Group | 0 days | 45±2°C for 6 months | -15±2°C for 6 months |
|---|---|---|---|
| Example 1 | 95% | 89% | 94% |
| Example 2 | 95% | 90% | 95% |
| Example 3 | 94% | 92% | 94% |
| Example 4 | 95% | 92% | 95% |
| Example 5 | 96% | 93% | 95% |
| Example 6 | 93% | 90% | 92% |
| Comparative Example 1 | 94% | 75% | 92% |
| Comparative Example 11 | 94% | 73% | 86% |
| Comparative Example 12 | 93% | 64% | 63% |
| Comparative Example 13 | 95% | 55% | 92% |
| Comparative Example 17 | 95% | 87% | 42% |
| Comparative Example 22 | 94% | 81% | 93% |
| Comparative Example 24 | 91% | 72% | 87% |
| Comparative Example 25 | 93% | 85% | 92% |

### Conclusion:

(1) Compared with the formula lacking any component of 3- o-ethyl ascorbic acid, the polyol, the nonionic surfactant and the anionic surfactant, using the formula provided by the present application is more beneficial to enhancing the state stability and content stability of the glabridin composition under high temperature and low temperature conditions.
(2) Compared with other nonionic surfactants, using oleth-20 and polyglycerol-10 oleate in the present application is more beneficial to enhancing the state stability and content stability of the glabridin composition under high temperature and low temperature conditions.
(3) Compared with other polyols, using 1,3-butanediol in the present application is more beneficial to enhancing the state stability and content stability of the glabridin composition under high temperature conditions.
(4) Compared with other oils, using caprylic acid/capric acid triglyceride in the present application is more beneficial to enhancing the state stability and content stability of the glabridin composition under high temperature conditions.
(5) Compared with other anionic surfactants, using sodium stearoyl glutamate in the present application is more beneficial to enhancing the state stability and content stability of the glabridin composition under high temperature and low temperature conditions.
(6) Compared with other preparation methods (for example, the addition sequence of 3-o-ethyl ascorbic acid or the addition sequence of water is different), using the preparation process provided by the present application is more beneficial to enhancing the state stability and content stability of the glabridin composition under high temperature and low temperature conditions.

### Experimental Example 4: Investigation of pH stability

Test method: According to "GB/T 13531.1-2008, General methods on determination of cosmetics-determination of pH", the glabridin compositions with high skin permeation, penetration-promoting and water solubility prepared in Examples 1-6, Comparative Example 1 and Comparative Example 22 were used respectively to determine the pH at each stable placement time point (see Experimental Example 3 for stable placement conditions and time), and test the pH stability of the samples. The results are shown in Table 19.

**Table 19: Investigation results of pH stability**

| Group | 0 days | 7 days | | 14 days | | 1 month | | 3 months | | 6 months | |
|---|---|---|---|---|---|---|---|---|---|---|---|
| | normal temperature | 45°C | -15° C | 45°C | -15°C | 45°C | -15°C | 45°C | -15°C | 45°C | -15°C |
| Example 1 | 5.61 | 5.59 | 5.60 | 5.56 | 5.58 | 5.52 | 5.57 | 5.41 | 5.49 | 5.26 | 5.42 |
| Example 2 | 5.54 | 5.52 | 5.55 | 5.51 | 5.51 | 5.46 | 5.51 | 5.35 | 5.40 | 5.24 | 5.35 |
| Example 3 | 5.58 | 5.55 | 5.56 | 5.50 | 5.54 | 5.42 | 5.53 | 5.26 | 5.43 | 5.11 | 5.39 |
| Example 4 | 5.72 | 5.68 | 5.72 | 5.64 | 5.71 | 5.52 | 5.69 | 5.43 | 5.53 | 5.32 | 5.40 |
| Example 5 | 5.62 | 5.58 | 5.61 | 5.50 | 5.59 | 5.42 | 5.58 | 5.26 | 5.47 | 4.85 | 5.38 |
| Example 6 | 5.68 | 5.64 | 5.65 | 5.60 | 5.64 | 5.56 | 5.62 | 5.50 | 5.52 | 5.23 | 5.46 |
| Comparative Example 1 | 5.66 | 5.60 | 5.64 | 5.58 | 5.62 | 5.55 | 5.58 | 5.48 | 5.52 | 5.34 | 5.44 |
| Comparative Example 22 | 5.58 | 5.42 | 5.52 | 5.25 | 5.48 | 4.91 | 5.30 | 4.54 | 5.10 | 4.21 | 4.86 |

Conclusion: According to Examples 1-6 and Comparative Example 22, compared with other preparation methods (for example, the addition sequence of 3-o-ethyl ascorbic acid is different), using the preparation process provided by the present application is more beneficial to enhancing the pH stability of the glabridin composition under high temperature and low temperature conditions.

### Experimental Example 5: Patch experiment

Test method: According to "Safety and technical standards for cosmetics (2015 Edition)-Human skin patch experiment", glabridin compositions of Examples 1-6 and Comparative Example 1 and Comparative Example 22 were used respectively to carry out the skin patch test (wherein the test samples were samples stored for 0 days, at 45°C for 6 months and at -15°C for 6 months respectively).

### Test scoring criteria: See Table 20.

Test result: See Table 21.

**Table 20: Scoring criteria**

| Ranking | Degree of reaction | Skin reaction |
|---|---|---|
| 0 | - | negative reaction |
| 1 | ± | suspicious reaction: only faint erythema |
| 2 | + | weak positive reaction: erythema, infiltration, edema and possibly papules |
| 3 | ++ | strong positive reaction: erythema, infiltration, edema, papules, herpes; the reaction may be beyond the test area |
| 4 | +++ | very strong positive reaction: obvious erythema, severe infiltration, edema, fusion herpes; the reaction is beyond the test area |

**Table 21: Test results**

| Group | Patch ranking | | |
|---|---|---|---|
| | 0 days | 45°C for 6 months | -15°C for 6 months |
| Example 1 | 0 | 0 | 0 |
| Example 2 | 0 | 0 | 0 |
| Example 3 | 0 | 0 | 0 |
| Example 4 | 0 | 0 | 0 |
| Example 5 | 0 | 0 | 0 |
| Example 6 | 0 | 0 | 0 |
| Comparative Example 1 | 0 | 2 | 1 |
| Comparative Example 22 | 0 | 3 | 2 |

### Conclusion:

(1) Compared with not adding 3-o-ethyl ascorbic acid, the formula provided by the present application is more beneficial to enhancing the safety of the glabridin composition, thus having unexpected technical effects.
(2) Compared with other preparation methods (for example, the addition sequence of 3-o-ethyl ascorbic acid is different), using the preparation process provided by the present application is more beneficial to enhancing the safety of the glabridin composition.

The method of the present application has been described through preferred examples, and relevant personnel can obviously modify or appropriately modify and combine the method and use described herein within the content and scope of the present application to achieve and apply the technology of the present application.

## Claims

1. A glabridin composition, wherein the glabridin composition is prepared from an S1 phase, an S2 phase and an S3 phase, in which the S1 phase consists of an oil and glabridin, the S2 phase is prepared from 3-o-ethyl ascorbic acid, an anionic surfactant, a nonionic surfactant, a polyol and water, and the S3 phase is water;
the nonionic surfactant is oleth-20 and polyglycerol-10 oleate;
the anionic surfactant is sodium stearoyl glutamate;
the oil is caprylic acid/capric acid triglyceride;
the polyol is 1,3-butanediol;
based on a total mass of the glabridin composition, the glabridin has a content of 0.10-4.00 wt%, and the 3-o-ethyl ascorbic acid has a content of 0.10-4.00 wt%; the nonionic surfactant has a total content of 0.70 wt%-10.00 wt%, and the oil has a content of 1.00 wt%-40.00 wt%; the polyol has a content of 0.60 wt%-6.00 wt%, the anionic surfactant has a content of 0.10 wt%-1.00 wt%, water in the S2 phase has a content of 0.30 wt%-3.00 wt%, and the balance is the S3 phase;
a preparation method of the glabridin composition comprises the following steps:
(1) mixing glabridin and the oil, heating for dissolution, and uniformly mixing to obtain the S1 phase;
(2) mixing 3-o-ethyl ascorbic acid, the anionic surfactant, the nonionic surfactant, the polyol and water, heating for dissolution, and uniformly mixing to obtain the S2 phase;
(3) adding the S1 phase obtained in the step (1) into the S2 phase obtained in the step (2) while stirring the S2 phase, and mixing, to form a gel; and
(4) then mixing the gel obtained in the step (3) with the S3 phase to obtain the glabridin composition.

2. The glabridin composition according to claim 1, wherein the oleth-20 has a content of 0.10 wt%-4.00 wt%, and the polyglycerol-10 oleate has a content of 0.60 wt%-6.00 wt%, based on the total mass of the glabridin composition.

3. The glabridin composition according to claim 1, wherein based on the total mass of the glabridin composition, the glabridin has a content of 1.00-4.00 wt%, and the 3-o-ethyl ascorbic acid has a content of 1.00-4.00 wt%; the oleth-20 has a total content of 0.50 wt%-4.00 wt%, the polyglycerol-10 oleate has a content of 6.00 wt%, and the oil has a content of 10.00 wt%-40.00 wt%; the polyol has a content of 6.00 wt%, the anionic surfactant has a content of 1.00 wt%, water in the S2 phase has a content of 3.00 wt%, and the balance is the S3 phase; or
based on the total mass of the glabridin composition, the glabridin has a content of 0.10 wt%, and the 3-o-ethyl ascorbic acid has a content of 0.10 wt%; the oleth-20 has a total content of 0.10 wt%, the polyglycerol-10 oleate has a content of 0.60 wt%, and the oil has a content of 1.00 wt%; the polyol has a content of 0.60 wt%, the anionic surfactant has a content of 0.10 wt%, water in the S2 phase has a content of 0.30 wt%, and the balance is the S3 phase.

4. The glabridin composition according to claim 1, wherein based on the total mass of the glabridin composition, the glabridin has a content of 1.00 wt%, and the 3-o-ethyl ascorbic acid has a content of 1.00 wt%; the oleth-20 has a total content of 0.50 wt% wt%, the polyglycerol-10 oleate has a content of 6.00 wt%, and the oil has a content of 10.00 wt%; the polyol has a content of 6.00 wt%, the anionic surfactant has a content of 1.00 wt%, water in the S2 phase has a content of 3.00 wt%, and the balance is the S3 phase; or
based on the total mass of the glabridin composition, the glabridin has a content of 1.00 wt%, and the 3-o-ethyl ascorbic acid has a content of 1.00 wt%; the oleth-20 has a total content of 1.00 wt%, the polyglycerol-10 oleate has a content of 6.00 wt%, and the oil has a content of 10.00 wt%; the polyol has a content of 6.00 wt%, the anionic surfactant has a content of 1.00 wt%, water in the S2 phase has a content of 3.00 wt%, and the balance is the S3 phase; or
based on the total mass of the glabridin composition, the glabridin has a content of 1.00 wt%, and the 3-o-ethyl ascorbic acid has a content of 2.00 wt%; the oleth-20 has a total content of 0.50 wt% wt%, the polyglycerol-10 oleate has a content of 6.00 wt%, and the oil has a content of 10.00 wt%; the polyol has a content of 6.00 wt%, the anionic surfactant has a content of 1.00 wt%, water in the S2 phase has a content of 3.00 wt%, and the balance is the S3 phase; or
based on the total mass of the glabridin composition, the glabridin has a content of 2.00 wt%, and the 3-o-ethyl ascorbic acid has a content of 2.00 wt%; the oleth-20 has a total content of 2.00 wt% wt%, the polyglycerol-10 oleate has a content of 6.00 wt%, and the oil has a content of 20.00 wt%; the polyol has a content of 6.00 wt%, the anionic surfactant has a content of 1.00 wt%, water in the S2 phase has a content of 3.00 wt%, and the balance is the S3 phase; or
based on the total mass of the glabridin composition, the glabridin has a content of 4.00 wt%, and the 3-o-ethyl ascorbic acid has a content of 4.00 wt%; the oleth-20 has a total content of 4.00 wt%, the polyglycerol-10 oleate has a content of 6.00 wt%, and the oil has a content of 40.00 wt%; the polyol has a content of 6.00 wt%, the anionic surfactant has a content of 1.00 wt%, water in the S2 phase has a content of 3.00 wt%, and the balance is the S3 phase.

5. The glabridin composition according to claim 1, wherein the heating for dissolution in the step (1) is heating to 60°C-80°C for dissolution.

6. The glabridin composition according to claim 1, wherein the heating for dissolution in the step (2) is heating to 60°C-70°C for dissolution.

7. The glabridin composition according to claim 1, wherein the heating for dissolution in the step (1) is heating to 70°C for dissolution; and the heating for dissolution in the step (2) is heating to 60°C for dissolution.

8. The glabridin composition according to claim 1, wherein a feeding mass ratio of water to the polyol in the step (2) is 1:1-1:2.

9. Use of the glabridin composition according to any one of claims 1 to 8 in preparation of cosmetics.

10. A cosmetic, comprising the glabridin composition according to any one of claims 1 to 8.

## Patentansprüche

1. Glabridin-Zusammensetzung, wobei die Glabridin-Zusammensetzung aus einer S1-Phase, einer S2-Phase und einer S3-Phase aufbereitet wird, wobei die S1-Phase aus Öl und Glabridin besteht, wobei die S2-Phase aus 3-O-Ethyl-Ascorbinsäure, einem anionischen Tensid, einem nichtionischen Tensid, einem Polyol und Wasser aufbereitet wird und die S3-Phase Wasser ist,
wobei das nichtionische Tensid Oleth-20 und Polyglycerin-10-Oleat ist,
wobei das anionische Tensid Natriumstearoylglutamat ist,
wobei das Öl Caprylsäure-/Caprinsäure-Triglycerid ist,
wobei das Polyol 1,3-Butandiol ist,
wobei bezogen auf eine Gesamtmasse der Glabridin-Zusammensetzung ein Gehalt an Glabridin 0,10 bis 4,00 Gew.-% und der Gehalt an 3-O-Ethyl-Ascorbinsäure 0,10 bis 4,00 Gew.-% beträgt, wobei ein Gesamtgehalt an nichtionischem Tensid 0,70 Gew.-% bis 10,00 Gew.-% und der Gehalt an Öl 1,00 Gew.-% bis 40,00 Gew.-% beträgt, wobei der Gehalt an Polyol 0,60 Gew.-% bis 6,00 Gew.-% und der Gehalt an anionischem Tensid 0,10 Gew.-% bis 1,00 Gew.-% beträgt, wobei ein Wassergehalt in der S2-Phase 0,30 Gew.-% bis 3,00 Gew.-% beträgt, und wobei der Rest die S3-Phase ist,
wobei ein Aufbereitungsverfahren für die Glabridin-Zusammensetzung folgende Schritte umfasst:
(1) Mischen von Glabridin und dem Öl, Erhitzen zum Auflösen und homogenes Vermischen, um die S1-Phase zu erhalten;
(2) Mischen von 3-O-Ethyl-Ascorbinsäure, dem anionischen Tensid, dem nichtionischen Tensid, dem Polyol und Wasser, Erhitzen zum Auflösen und homogenes Vermischen, um die S2-Phase zu erhalten;
(3) Zugeben der in Schritt (1) erhaltenen S1-Phase zu der in Schritt (2) erhaltenen S2-Phase unter Rühren der S2-Phase und Mischen, wodurch ein Gel gebildet wird; und
(4) anschließend Mischen des in Schritt (3) erhaltenen Gels mit der S3-Phase, um die Glabridin-Zusammensetzung zu erhalten.

2. Glabridin-Zusammensetzung nach Anspruch 1, wobei bezogen auf die Gesamtmasse der Glabridin-Zusammensetzung der Gehalt an Oleth-20 0,10 Gew.-% bis 4,00 Gew.-% und der Gehalt an Polyglycerin-10-Oleat 0,60 Gew.-% bis 6,00 Gew.-% beträgt.

3. Glabridin-Zusammensetzung nach Anspruch 1, wobei bezogen auf die Gesamtmasse der Glabridin-Zusammensetzung der Gehalt an Glabridin 1,00 bis 4,00 Gew.-% und der Gehalt an 3-O-Ethyl-Ascorbinsäure 1,00 bis 4,00 Gew.-% beträgt, wobei der Gesamtgehalt an Oleth-20 0,50 Gew.-% bis 4,00 Gew.-% beträgt, wobei der Gehalt an Polyglycerin-10-Oleat 6,00 Gew.-% beträgt, wobei der Gehalt an Öl 10,00 Gew.-% bis 40,00 Gew.-% beträgt, wobei der Gehalt an Polyol 6,00 Gew.-% beträgt, wobei der Gehalt an anionischem Tensid 1,00 Gew.-% beträgt, wobei der Wassergehalt in der S2-Phase 3,00 Gew.-% beträgt, und wobei der Rest die S3-Phase ist; oder
wobei bezogen auf die Gesamtmasse der Glabridin-Zusammensetzung der Gehalt an Glabridin 0,10 Gew.-% und der Gehalt an 3-O-Ethyl-Ascorbinsäure 0,10 Gew.-% beträgt, wobei der Gesamtgehalt an Oleth-20 0,10 Gew.-% beträgt, wobei der Gehalt an Polyglycerin-10-Oleat 0,60 Gew.-% beträgt, wobei der Gehalt an Öl 1,00 Gew.-% beträgt, wobei der Gehalt an Polyol 0,60 Gew.-% beträgt, wobei der Gehalt an anionischem Tensid 0,10 Gew.-% beträgt, wobei der Wassergehalt in der S2-Phase 0,30 Gew.-% beträgt, und wobei der Rest die S3-Phase ist.

4. Glabridin-Zusammensetzung nach Anspruch 1, wobei bezogen auf die Gesamtmasse der Glabridin-Zusammensetzung der Gehalt an Glabridin 1,00 Gew.-% und der Gehalt an 3-O-Ethyl-Ascorbinsäure 1,00 Gew.-% beträgt, wobei der Gesamtgehalt an Oleth-20 0,50 Gew.-% beträgt, wobei der Gehalt an Polyglycerin-10-Oleat 6,00 Gew.-% beträgt, wobei der Gehalt an Öl 10,00 Gew.-% beträgt, wobei der Gehalt an Polyol 6,00 Gew.-% beträgt, wobei der Gehalt an anionischem Tensid 1,00 Gew.-% beträgt, wobei der Wassergehalt in der S2-Phase 3,00 Gew.-% beträgt, und wobei der Rest die S3-Phase ist; oder
wobei bezogen auf die Gesamtmasse der Glabridin-Zusammensetzung der Gehalt an Glabridin 1,00 Gew.-% und der Gehalt an 3-O-Ethyl-Ascorbinsäure 1,00 Gew.-% beträgt, wobei der Gesamtgehalt an Oleth-20 1,00 Gew.-% beträgt, wobei der Gehalt an Polyglycerin-10-Oleat 6,00 Gew.-% beträgt, wobei der Gehalt an Öl 10,00 Gew.-% beträgt, wobei der Gehalt an Polyol 6,00 Gew.-% beträgt, wobei der Gehalt an anionischem Tensid 1,00 Gew.-% beträgt, wobei der Wassergehalt in der S2-Phase 3,00 Gew.-% beträgt, und wobei der Rest die S3-Phase ist; oder
wobei bezogen auf die Gesamtmasse der Glabridin-Zusammensetzung der Gehalt an Glabridin 1,00 Gew.-% und der Gehalt an 3-O-Ethyl-Ascorbinsäure 2,00 Gew.-% beträgt, wobei der Gesamtgehalt an Oleth-20 0,50 Gew.-% beträgt, wobei der Gehalt an Polyglycerin-10-Oleat 6,00 Gew.-% beträgt, wobei der Gehalt an Öl 10,00 Gew.-% beträgt, wobei der Gehalt an Polyol 6,00 Gew.-% beträgt, wobei der Gehalt an anionischem Tensid 1,00 Gew.-% beträgt, wobei der Wassergehalt in der S2-Phase 3,00 Gew.-% beträgt, und wobei der Rest die S3-Phase ist; oder
wobei bezogen auf die Gesamtmasse der Glabridin-Zusammensetzung der Gehalt an Glabridin 2,00 Gew.-% und der Gehalt an 3-O-Ethyl-Ascorbinsäure 2,00 Gew.-% beträgt, wobei der Gesamtgehalt an Oleth-20 2,00 Gew.-% beträgt, wobei der Gehalt an Polyglycerin-10-Oleat 6,00 Gew.-% beträgt, wobei der Gehalt an Öl 20,00 Gew.-% beträgt, wobei der Gehalt an Polyol 6,00 Gew.-% beträgt, wobei der Gehalt an anionischem Tensid 1,00 Gew.-% beträgt, wobei der Wassergehalt in der S2-Phase 3,00 Gew.-% beträgt, und wobei der Rest die S3-Phase ist; oder
wobei bezogen auf die Gesamtmasse der Glabridin-Zusammensetzung der Gehalt an Glabridin 4,00 Gew.-% und der Gehalt an 3-O-Ethyl-Ascorbinsäure 4,00 Gew.-% beträgt, wobei der Gesamtgehalt an Oleth-20 4,00 Gew.-% beträgt, wobei der Gehalt an Polyglycerin-10-Oleat 6,00 Gew.-% beträgt, wobei der Gehalt an Öl 40,00 Gew.-% beträgt, wobei der Gehalt an Polyol 6,00 Gew.-% beträgt, wobei der Gehalt an anionischem Tensid 1,00 Gew.-% beträgt, wobei der Wassergehalt in der S2-Phase 3,00 Gew.-% beträgt, und wobei der Rest die S3-Phase ist.

5. Glabridin-Zusammensetzung nach Anspruch 1, wobei das Erhitzen zum Auflösen in Schritt (1) ein Erhitzen auf 60 °C bis 80 °C zum Auflösen ist.

6. Glabridin-Zusammensetzung nach Anspruch 1, wobei das Erhitzen zum Auflösen in Schritt (2) ein Erhitzen auf 60 °C bis 70 °C zum Auflösen ist.

7. Glabridin-Zusammensetzung nach Anspruch 1, wobei das Erhitzen zum Auflösen in Schritt (1) ein Erhitzen auf 70 °C zum Auflösen ist, wobei das Erhitzen zum Auflösen in Schritt (2) ein Erhitzen auf 60 °C zum Auflösen ist.

8. Glabridin-Zusammensetzung nach Anspruch 1, wobei ein Einsatzmassenverhältnis von Wasser zu dem Polyol in Schritt (2) 1:1 bis 1:2 beträgt.

9. Anwendung der Glabridin-Zusammensetzung nach einem der Ansprüche 1 bis 8 bei der Aufbereitung von Kosmetika.

10. Kosmetikum, umfassend die Glabridin-Zusammensetzung nach einem der Ansprüche 1 bis 8.

## Revendications

1. Composition de glabridine, dans laquelle la composition de glabridine est préparée à partir d'une phase S1, d'une phase S2 et d'une phase S3, dans laquelle la phase S1 est constituée d'huile et de glabridine, la phase S2 est préparée à partir d'acide 3-o-éthyle-ascorbique, d'un tensioactif anionique, d'un tensioactif non ionique, d'un polyol et d'eau, et la phase S3 est de l'eau ;
le tensioactif non ionique est de l'oleth-20 et de l'oléate de polyglycérol-10 ;
le tensioactif anionique est du stéaroylglutamate de sodium ;
l'huile est un triglycéride d'acide caprylique/acide caprique ;
le polyol est du 1,3-butanediol ;
sur la base d'une masse totale de la composition de glabridine, la glabridine a une teneur de 0,10 à 4,00 % en poids, et l'acide 3-o-éthyle-ascorbique a une teneur de 0,10 à 4,00 % en poids ; le tensioactif non ionique a une teneur totale de 0,70 % en poids à 10,00 % en poids, et l'huile a une teneur de 1,00 % en poids à 40,00 % en poids ; le polyol a une teneur de 0,60 % en poids à 6,00 % en poids, le tensioactif anionique a une teneur de 0,10 % en poids à 1,00 % en poids, de l'eau dans la phase S2 a une teneur de 0,30 % en poids à 3,00 % en poids, et le reste est la phase S3 ;
un procédé de préparation de la composition de glabridine comprend les étapes suivantes consistant à :
(1) mélanger la glabridine et l'huile, chauffer pour la dissolution, puis mélanger uniformément afin d'obtenir la phase S1 ;
(2) mélanger l'acide 3-o-éthyle-ascorbique, le tensioactif anionique, le tensioactif non ionique, le polyol et l'eau, chauffer pour la dissolution, puis mélanger uniformément afin d'obtenir la phase S2 ;
(3) ajouter la phase S1 obtenue à l'étape (1) à la phase S2 obtenue à l'étape (2) tout en agitant la phase S2, puis mélanger, afin de former un gel ; et
(4) mélanger ensuite le gel obtenu à l'étape (3) avec la phase S3 afin d'obtenir la composition de glabridine.

2. Composition de glabridine selon la revendication 1, dans laquelle l'oleth-20 a une teneur de 0,10 % en poids à 4,00 % en poids, et l'oléate de polyglycérol-10 a une teneur de 0,60 % en poids à 6,00 % en poids, sur la base de la masse totale de la composition de glabridine.

3. Composition de glabridine selon la revendication 1, dans laquelle, sur la base de la masse totale de la composition de glabridine, la glabridine a une teneur de 1,00 et 4,00 % en poids, et l'acide 3-o-éthyle-ascorbique a une teneur de 1,00 et 4,00 % en poids ; l'oleth-20 a une teneur totale de 0,50 % en poids à 4,00 % en poids, l'oléate de polyglycérol-10 a une teneur de 6,00 % en poids, et l'huile a une teneur de 10,00 % en poids à 40,00 % en poids ; le polyol a une teneur de 6,00 % en poids, le tensioactif anionique a une teneur de 1,00 % en poids, l'eau dans la phase S2 a une teneur de 3,00 % en poids, et le reste est la phase S3 ; ou
sur la base de la masse totale de la composition de glabridine, la glabridine a une teneur de 0,10 % en poids, et l'acide 3-o-éthyle-ascorbique a une teneur de 0,10 % en poids ; l'oleth-20 a une teneur totale de 0,10 % en poids, l'oléate de polyglycérol-10 a une teneur de 0,60 % en poids, et l'huile a une teneur de 1,00 % en poids ; le polyol a une teneur de 0,60 % en poids, le tensioactif anionique a une teneur de 0,10 % en poids, de l'eau dans la phase S2 a une teneur de 0,30 % en poids, et le reste est la phase S3.

4. Composition de glabridine selon la revendication 1, dans laquelle, sur la base de la masse totale de la composition de glabridine, la glabridine a une teneur de 1,00 % en poids, et l'acide 3-o-éthyle-ascorbique a une teneur de 1,00 % en poids ; l'oleth-20 a une teneur totale de 0,50 % en poids, l'oléate de polyglycérol-10 a une teneur de 6,00 % en poids, et l'huile a une teneur de 10,00 % en poids ; le polyol a une teneur de 6,00 % en poids, le tensioactif anionique a une teneur de 1,00 % en poids, l'eau dans la phase S2 a une teneur de 3,00 % en poids, et le reste est la phase S3 ; ou
sur la base de la masse totale de la composition de glabridine, la glabridine a une teneur de 1,00 % en poids, et l'acide 3-o-éthyle-ascorbique a une teneur de 1,00 % en poids ; l'oleth-20 a une teneur totale de 1,00 % en poids,
l'oléate de polyglycérol-10 a une teneur de 6,00 % en poids, et l'huile a une teneur de 10,00 % en poids ; le polyol a une teneur de 6,00 % en poids, le tensioactif anionique a une teneur de 1,00 % en poids, de l'eau dans la phase S2 a une teneur de 3,00 % en poids, et le reste est la phase S3 ; ou
sur la base de la masse totale de la composition de glabridine, la glabridine a une teneur de 1,00 % en poids, et l'acide 3-o-éthyle-ascorbique a une teneur de 2,00 % en poids ; l'oleth-20 a une teneur totale de 0,50 % en poids, l'oléate de polyglycérol-10 a une teneur de 6,00 % en poids, et l'huile a une teneur de 10,00 % en poids ; le polyol a une teneur de 6,00 % en poids, le tensioactif anionique a une teneur de 1,00 % en poids, l'eau dans la phase S2 a une teneur de 3,00 % en poids, et le reste est la phase S3 ; ou
sur la base de la masse totale de la composition de glabridine, la glabridine a une teneur de 2,00 % en poids, et l'acide 3-o-éthyle-ascorbique a une teneur de 2,00 % en poids ; l'oleth-20 a une teneur totale de 2,00 % en poids, l'oléate de polyglycérol-10 a une teneur de 6,00 % en poids, et l'huile a une teneur de 20,00 % en poids ; le polyol a une teneur de 6,00 % en poids, le tensioactif anionique a une teneur de 1,00 % en poids, l'eau dans la phase S2 a une teneur de 3,00 % en poids, et le reste est la phase S3 ; ou
sur la base de la masse totale de la composition de glabridine, la glabridine a une teneur de 4,00 % en poids, et l'acide 3-o-éthyle-ascorbique a une teneur de 4,00 % en poids ; l'oleth-20 a une teneur totale de 4,00 % en poids, l'oléate de polyglycérol-10 a une teneur de 6,00 % en poids, et l'huile a une teneur de 40,00 % en poids ; le polyol a une teneur de 6,00 % en poids, le tensioactif anionique a une teneur de 1,00 % en poids, l'eau dans la phase S2 a une teneur de 3,00 % en poids, et le reste est la phase S3.

5. Composition de glabridine selon la revendication 1, dans laquelle le chauffage pour la dissolution à l'étape (1) consiste à chauffer à une température de 60 °C et 80 °C pour la dissolution.

6. Composition de glabridine selon la revendication 1, dans laquelle le chauffage pour la dissolution à l'étape (2) consiste à chauffer à une température de 60 °C et 70 °C pour la dissolution.

7. Composition de glabridine selon la revendication 1, dans laquelle le chauffage pour la dissolution à l'étape (1) consiste à chauffer à 70 °C pour la dissolution ; et le chauffage pour la dissolution à l'étape (2) consiste à chauffer à 60 °C pour la dissolution.

8. Composition de glabridine selon la revendication 1, dans laquelle un rapport massique d'alimentation entre de l'eau et le polyol à l'étape (2) est de 1:1 à 1:2.

9. Utilisation de la composition de glabridine selon l'une quelconque des revendications 1 à 8 pour la préparation de produits cosmétiques.

10. Produit cosmétique comprenant la composition de glabridine selon l'une quelconque des revendications 1 à 8.
